# EUROPÄISCHE PATENTANMELDUNG

(11) **EP 3 685 808 A1**
(43) Veröffentlichungstag der Anmeldung: **29.07.2020**
(21) Anmeldenummer: 19152967.6
(22) Anmeldetag: 22.01.2019
(51) Int. Cl.: A61F 5/00, A61M 39/08, A61M 25/00, A61B 1/06, A61B 90/30, A61B 17/00

(54) **GASTRISCHER KALIBRIERSCHLAUCH**

(71) Anmelder: Q Medical International AG, 8260 Stein am Rhein (CH)
(72) Erfinder: Desombre, Rainer, 40667 Meerbusch (DE)
(74) Vertreter: dompatent von Kreisler Selting Werner - Partnerschaft von Patent- und Rechtsanwälten mbB

(57) **Zusammenfassung**

Bei einem gastrische Kalibrierschläuche (1) mit einem länglichen flexiblen Hohlkörper (2), der eine Außenwand (4) und ein erstes und ein zweites Schlauchende (7, 14) aufweist, ist vorgesehen, dass der längliche flexible Hohlkörper (2) zumindest zwei Kanäle (6, 8) aufweist, die sich jeweils in Längsrichtung des Hohlkörpers (2) erstrecken, wobei der erste Kanal (6) im Bereich des zweiten Schlauchendes (14) zumindest eine Öffnung (22, 18) aufweist und wobei der zweiten Kanal (8) am zweiten Schlauchende (14) geschlossen ist, wobei zumindest ein Leuchtmit-tel (30, 32) im Bereich des zweiten Schlauendes (14) im zweiten Kanal (8) angeordnet ist.

## Beschreibung

Die Erfindung betrifft einen gastrischen Kalibrierschlauch nach dem Oberbegriff des Anspruchs 1.

Aus dem Stand der Technik sind gastrische Kalibrierschläuche bekannt, die bei chirurgischen Operationsverfahern, insbesondere bei Eingriffen am Magen und bei bariatrischen Eingriffen wie Magenresektionen, Laparoskopische Banded Sleeve-Gastrektomie (LBSG), die konventionelle Sleeve-Gastrektomie (LCSG), Fundoplicatio, Magenbandeinbringungen und beispielweise Magen-Bypassen verwendet werden können. Der gastrische Kalibrierschlauch dient der sichtbaren und fühlbaren Abgrenzung von Teilen des Magens sowie der Dekomprimierung, Entleerung und Entfernung von Magensäure.

Der gastrische Kalibrierschlauch weist einen länglichen flexiblen Hohlkörper auf, der eine Außenwand und ein erstes und ein zweites Schlauchende aufweist. Das zweite Schlauchende kann über den Mund und die Speiseröhre in den Magen eingeführt werden. Das erste Schlauchende verbleibt dabei außerhalb des Körpers. An das erste Schlauchende können medizinische Geräte angekoppelt werden, wie beispielsweise Spritzen, Kanülen oder Einrichtungen, die Luft oder Flüssigkeiten in den Kalibrierschlauch einführen oder Unterdruck im Kalibrierschlauch erzeugen können.

Es besteht zunehmend Bedarf, dass der behandelnde bzw. die Operation durchführende Arzt insbesondere im Hinblick auf die sichtbare und führbare Abgrenzung von Teilen des Magens unterstützt wird.

Aufgabe der vorliegenden Erfindung ist es daher, einen gastrischen Kalibrierschlauch zu schaffen, der den behandelnde bzw. die Operation durchführende Arzt bei der sichtbaren oder führbaren Abgrenzung von Teilen des Magens unterstützt.

Zur Lösung dieser Aufgabe dienen die Merkmale des Anspruchs 1.

Die Erfindung sieht in vorteilhafter Weise vor, dass der längliche flexible Hohlkörper zumindest zwei Kanäle aufweist, die sich jeweils in Längsrichtung des Hohlkörpers erstrecken, wobei der erste Kanal im Bereich des zweiten Schlauchendes zumindest eine Öffnung aufweist und wobei der zweite Kanal am zweiten Schlauchende geschlossen ist, wobei zumindest ein Leuchtmittel im Bereich des zweiten Schlauendes im zweiten Kanal angeordnet ist.

Die vorliegende Erfindung hat den Vorteil, dass ein Leuchtmittel in dem gastrischen Kalibrierschlauch im Bereich des zweiten Schlauchendes angeordnet ist und dadurch bei einer Operation die Position des Kalibrierschlauchs wesentlich besser sichtbar ist und dem behandelnden bzw. die Operation durchführenden Arzt bei der Abgrenzung von Teilen des Magens hilft.

Ferner ist durch die Einteilung des Hohlkörpers in mindestens zwei Kanäle das Leuchtmittel innerhalb des zweiten Kanals sehr gut geschützt und behindert nicht die Absaugung in dem ersten Kanal.

Der flexible Hohlkörper ist länglich ausgeführt und besitzt eine Längsachse. Der Hohlkörper ist somit in einer Richtung parallel zur Längsachse länger als er in einer Richtung quer zur Längsachse breit ist.

Es wird in der vorliegenden Erfindung der Begriff "im Bereich von" dem zweiten Schlauchende verwendet, dabei ist der Begriff so zu verstehen, dass dieser Bereich zumindest die Hälfte des Kalibrierschlauchs umfasst, die auch das zweite Schlauchende umfasst. Vorzugsweise ist "im Bereich von" so zu verstehen, dass lediglich 1/3, insbesondere ¼, besonders bevorzugt 1/5 des Kalibrierschlauchs umfasst, das das jeweilige Schlauchende beinhaltet.

Bei der Verwendung des Begriffs im Bereich des ersten Schlauchendes gilt entsprechendes.

Es kann eine Stromversorgungsvorrichtung für das zumindest eine Leuchtmittel vorgesehen sein, wobei die Stromversorgungseinrichtung mit dem Leuchtmittel über zumindest ein Stromkabel verbunden ist, wobei die Stromversorgungsvorrichtung außerhalb des Hohlkörpers angeordnet ist.

Dadurch, dass die Stromversorgungsvorrichtung außerhalb des Hohlkörpers angeordnet ist, kann die Geometrie der Stromversorgungsvorrichtung völlig flexibel ausgeführt werden.

Die Stromversorgungsvorrichtung kann von außen an der Außenwand des Hohlkörpers mit dem Hohlkörper verbunden sein.

Auf diese Weise hat die Stromversorgungseinrichtung einen festgelegten Platz und behindert bei der Operation nicht.

Die Stromversorgungsvorrichtung kann an dem Hohlkörper lösbar befestigt sein, vorzugsweise mittels einer Klippverbindung mit dem Hohlkörper befestigt sein.

Der zweite Kanal kann zumindest teilweise durch die Außenwand des Hohlkörpers begrenzt sein, wobei die den zweiten Kanal begrenzende Außenwand im Bereich des ersten Schlauchendes eine Austrittsöffnung aufweist, durch die das zumindest eine Stromkabel geführt ist, das die Stromversorgungsvorrichtung und das Leuchtmittel verbindet.

Das ersten Schlauchende wird bei einer Operation nicht in den Patienten eingeführt und deshalb kann in diesem Bereich die außerhalb des Hohlkörpers angeordneten Stromversorgungsvorrichtung angeordnet sein. Dadurch dass das Stromkabel in diesem Bereich aus dem zweiten Kanal herausgeführt wird, behindert es bei der Operation nicht.

Das zumindest eine Leuchtmittel kann ein erstes LED Band sein, das eine Oberseite und eine Unterseite aufweist, wobei auf der Oberseite mehrere nebeneinander angeordnete LEDs angeordnet sind.

Bei einem solchen LED Band mit mehreren LEDs ist eine sehr hohe Lichtstärke sichergestellt. Der Lichtstrom beträgt 4000 bis 5000 Lumen.

Es können auch unterschiedliche LEDs auf dem LED Band angeordnet sein, so dass unterschiedliche Lichtfarben eingestellt werden können.

Es kann ein zweites Leuchtmittel vorgesehen sein, das ein zweites LED Band ist, das eine Oberseite und eine Unterseite aufweist, wobei auf der Oberseite mehrere nebeneinander angeordnete LEDs angeordnet sind und wobei die Unterseite des zweiten LED Bandes mit der Unterseite des ersten LED Bandes verbunden ist, so dass die auf den jeweiligen Oberseiten angeordneten LEDs in entgegengesetzte Richtungen leuchten.

Dies hat den Vorteil, dass zwei Seiten sehr gut beleuchtet werden und somit der Raum rund um den Kalibrierschlauch sehr gut ausgeleuchtet ist.

Alternativ können auf der Unterseite des ersten LED Bandes ebenfalls mehrere nebeneinander angeordnete LED vorgesehen sein. Auch bei einer solchen Ausgestaltung würde Licht in zwei Richtungen ausgestrahlt werden.

Das erste und/oder zweite LED Band können vorzugsweise zwischen 2 und 30 cm lang sein.

Am zweiten Schlauchende kann ein Abschlusselement vorgesehen sein, das den Hohlkörper abschließt.

Das Abschlusselement kann stumpf ausgebildet sein. Auf diese Weise lässt sich der gastrische Kalibrierschlauch einfach über die Speiseröhre in den Magen ohne Verletzung von Gewebe einführen.

Das Abschlusselement kann ein Material aufweisen, das radiopakes Material, vorzugsweise Bariumsulfat umfasst.

Auf diese Weise kann das Abschlusselement z.B. mit Röntgengeräten lokalisiert werden. Dies kann sicherstellen, dass die Kalibrierschläuche nicht bei einer Operation im Körper verloren gehen.

Die Stromversorgungsvorrichtung kann an- und ausgeschaltet werden. Die Stromversorgungsvorrichtung kann somit einen Schalter umfassen.

Die Stromversorgungseinrichtung kann zumindest eine Batterie und/oder einen Akku aufweisen. Die Batterien können beispielsweise handelsübliche AA-Batterien sein.

Dadurch, dass die Stromversorgungsvorrichtung außerhalb des Hohlkörpers angeordnet ist und die Stromversorgungsvorrichtung eine beliebige Geometrie aufweisen kann, können auch handelsübliche Batterien verwendet werden.

Die Außenwand des Hohlkörpers und/oder Begrenzungswand zwischen den zumindest zwei Kanälen kann aus einem Material bestehen, das Silikon umfasst.

In der vorliegenden Erfindung wird der Begriff flexible Hohlkörper verwendet. Flexibel bedeutet in diesem Zusammenhang, dass der Hohlkörper gebogen werden kann. Vorzugsweise sollte dieser um mindestens 90° gebogen werden können.

Im Folgenden wird unter Bezugnahme auf die Zeichnungen Ausführungsbeispiele der vorliegenden Erfindung näher erläutert.

Es zeigen schematisch:
- Fig. 1: einen gastrischen Kalibrierschlauch gemäß der vorliegenden Erfindung,
- Fig. 2: eine etwas deutlicheren Darstellung des Kalibrierschlauchs aus Fig.1,
- Fig. 3: eine Querschnittsansicht des Kalibrierschlauchs aus Fig.1,
- Fig. 4: eine alternative Ausgestaltung eines Kalibrierschlauchs im Querschnitt,
- Fig. 5: die Stromversorgungsvorrichtung mit Halterung aus Fig. 1,
- Fig. 6: einen Verschluss-Schieber aus Fig. 1.

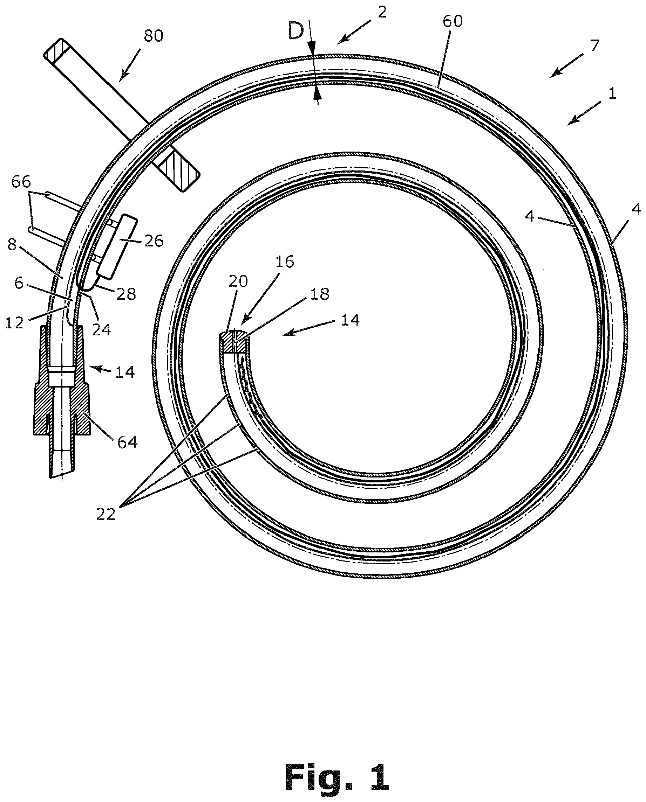

Fig. 1 zeigt einen gastrischen Kalibrierschlauch 1. Der gastrische Kalibrierschlauch 1 weist einen länglichen flexiblen Hohlkörper 2 auf. Der längliche flexible Hohlkörper 2 weist eine Längsachse 60 auf. Ferner weist der Hohlkörper 2 eine Außenwand 4 auf. Der Hohlkörper 2 weist ein erstes Schlauchende 7 und ein zweites Schlauchende 14 auf. Das zweite Schlauchende 14 kann bei einer Operation über den Mund und die Speiseröhre in den Magen eingeführt werden. Das erste Schlauchende 7 verbleibt dabei außerhalb des Körpers.

Der gastrische Kalibrierschlauch 1 kann bei chirurgischen Operationsverfahren, insbesondere bei Eingriffen am Magen und bei bariatrischen Eingriffen wie Magenresektionen, Laparoskopische Banded Sleeve-Gastrektomie (LBSG), konventionelle Sleeve-Gastrektomie (LCSG), Fundoplicatio, Magenbandeinbringungen und beispielweise Magen-Bypassen verwendet werden. Der gastrische Kalibrierschlauch 1 kann der sichtbaren und fühlbaren Abgrenzung von Teilen des Magens sowie der Dekomprimierung, Entleerung und Entfernung von Magensäure dienen.

Der Kalibrierschlauch 1 weist am zweiten Schlauchende 14 ein Abschlusselement 16 auf. Das Abschlusselement 16 ist stumpf ausgebildet, so dass es beim Einführen des zweiten Schlauchendes 14 in den menschlichen Körper nicht zu Verletzungen kommen kann. Der Hohlkörper 2 weist zumindest zwei Kanäle 6 und 8 auf. Die Kanäle 6 und 8 erstrecken sich in Längsrichtung des Hohlkörpers 2.

Der erste Kanal 6 weist im Bereich des zweiten Schlauchendes 14 zumindest eine Öffnung 22 auf. Im vorliegenden Fall sind mehrere Öffnungen 22 vorgesehen. Ferner weißt auch das Abschlusselement 16 eine Öffnung 18 auf, die in den ersten Kanal 6 einmündet.

Der zweite Kanal 8 ist am zweiten Schlauchende 14 geschlossen ausgebildet. Das Abschlusselement 16 schließt am zweiten Schlauchende 14 den zweiten Kanal 8 ab. Im Bereich des zweiten Kanals 8 ist in dem Abschlusselement 16 keine Öffnung vorgesehen. Im Bereich des zweiten Schlauchendes 14 weist der zweite Kanal 8 zumindest ein Leuchtmittel 30 auf.

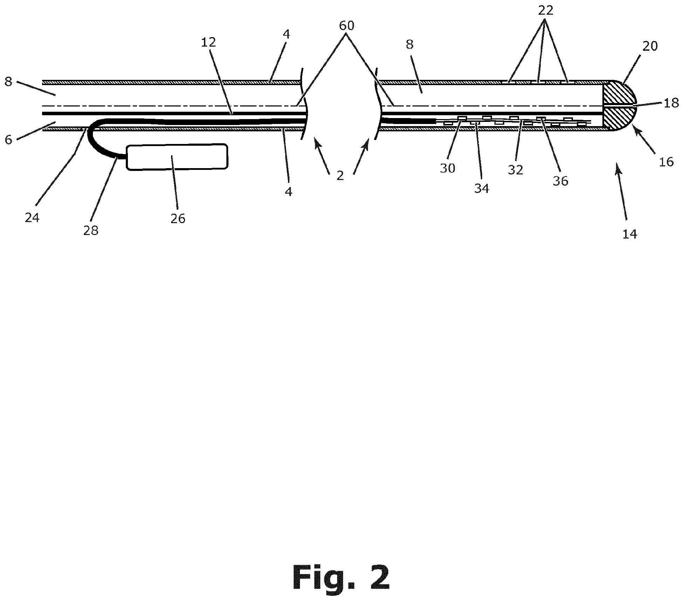

Im vorliegenden Ausführungsbeispiel sind ein erstes und ein zweites Leuchtmittel vorgesehen, die bei der Beschreibung zu Fig. 2 noch näher erläutert werden. Alternativ kann auch lediglich ein Leuchtmittel vorgesehen sein.

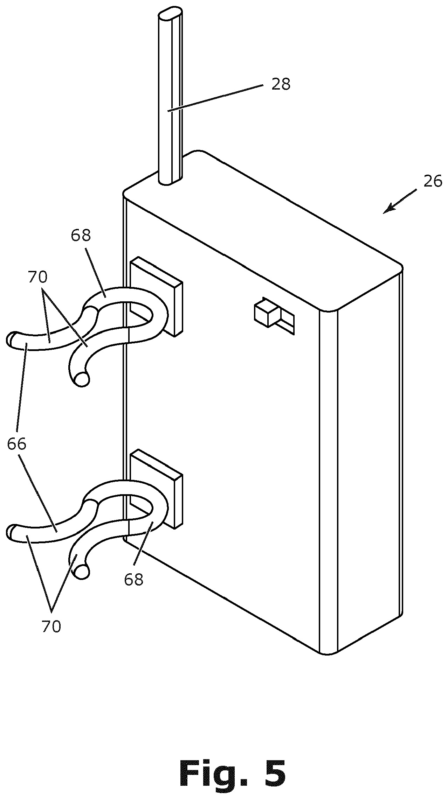

Die Leuchtmittel 30, 32 sind über ein Stromkabel 28 mit einer Stromversorgungsvorrichtung 26 verbunden. Die Stromversorgungsvorrichtung 26 ist außerhalb des Hohlkörpers 2 angeordnet. Die Stromversorgungsvorrichtung 26 ist außen an der Außenwand 4 des Hohlkörpers 2 mit dem Hohlkörper 2 verbunden. Die Stromversorgungsvorrichtung 26 kann lösbar über eine in Fig. 5 näher dargestellte Klippverbindung mit dem Hohlkörper 2 verbunden sein.

Das Stromkabel 28 verläuft durch den zweiten Kanal 8 und tritt am ersten Schlauchende 7 durch eine Austrittsöffnung 24 aus dem zweiten Kanal 8 und damit aus dem Hohlkörper 2 aus und ist zu der Stromversorgungsvorrichtung 26 geführt.

Die Stromversorgungsvorrichtung 26 kann eine Batterie oder einen Akku aufweisen, um das zumindest eine Leuchtmittel 20, 32 mit Strom zu versorgen. An der Stromversorgungsvorrichtung 26 kann ein Schalter vorgesehen sein, mit dem die Leuchtmittel an- und ausgeschaltet werden können.

Am ersten Schlauchende 7 kann der zweite Kanal geschlossen oder offen ausgebildet sein. Im vorliegenden Fall ist der zweite Kanal 8 am ersten Schlauchende 7 geschlossen.

Am ersten Schlauchende 7 können an den ersten Kanal 6 medizinische Geräte angekoppelt werden, wie beispielsweise Spritzen, Kanülen oder Einrichtungen, die Luft oder Flüssigkeiten in den ersten Kanal 6 des Kalibrierschlauchs 1 einführen oder einen Unterdruck im ersten Kanal 6 des Kalibrierschlauchs 1 erzeugen können. Sofern in dem ersten Kanal 6 ein Unterdruck erzeugt wird, liegt auch an den Öffnungen 22 und 18 ein Unterdruck an. Auf diese Weise kann mittels des ersten Kanals 6 eine Absaugung stattfinden.

In Fig. 1 ist eine Verbindungsvorrichtung 64 vorgesehen, das mit dem ersten Schlauchende verbunden ist, wobei der Kalibrierschlauch 1 in die Verbindungsvorrichtung 64 eingesteckt ist. Mittels der Verbindungsvorrichtung 64 kann eine einfache und sichere Ankopplung von unterschiedlichen medizinischen Geräten mit unterschiedlichsten Anschlussgeometrien gewährleistet werden.

An dem dem Kalibrierschlauch 1 abgewandten Ende der Verbindungsvorrichtung weist die Verbindungsvorrichtung zur Ankopplung von unterschiedlichen medizinischen Geräten zumindest eine zweite sich verjüngende Vertiefung und zumindest eine, die zweite Vertiefung ringförmig umgebende sich verjüngende Ausnehmung auf, so dass die unterschiedlichen medizinischen Geräte wahlweise in die zweite sich verjüngende Vertiefung oder in die sich verjüngende Ausnehmung zum Ankoppeln einsteckbar sind.

Der erfindungsgemäße Kalibrierschlauch kann jedoch auch ohne oder mit einer anderen Verbindungsvorrichtung mit einem medizinischen Gerät verbunden sein.

In Fig. 2 ist der Kalibrierschlauch detaillierter dargestellt, wobei zur Verdeutlichung das erste Schlauchende 7 und das zweite Schlauchende 14 näher dargestellt sind und der mittlere Bereich des Hohlkörpers 2 ausgespart sind. Ferner sind in Fig.2 auch die Verbindungsvorrichtung 64 und der Verschuss-Schieber nicht dargestellt.

Wie Fig. 2 zu entnehmen ist, ist der erste Kanal 6 und der zweite Kanal 8 durch eine Begrenzungswand 12 voneinander getrennt. Die Begrenzungswand 12 kann das gleiche Material aufweisen wie die Außenwand 4 des Hohlkörpers 2.

Der zweiten Kanal 8 ist auch zumindest teilweise durch die Außenwand 4 des Hohlkörpers begrenzt. In dieser den zweiten Kanal 8 begrenzenden Außenwand 4 ist am ersten Schlauchende 7 eine Austrittsöffnung 24 vorgesehen, durch die das Stromkabel 28 geführt ist.

Auch in Fig. 2 ist die Stromversorgungsvorrichtung 26 zu erkennen, die außerhalb des Hohlkörpers 2 angeordnet ist und mit diesem über eine vorzugsweise lösbare Klippverbindung verbunden ist.

Ferner kann aus Fig. 2 entnommen werden, dass am zweiten Schlauchende 14 ein erstes und ein zweites Leuchtmittel 30, 32 angeordnet sind. Die Leuchtmittel 30, 32 können, wie dargestellt, als LED Bänder ausgeführt sein.

Das erste und das zweite LED Band weisen jeweils eine Oberseite auf, auf denen die jeweiligen LED 34, 36 angeordnet sind. Die LED Bänder sind mit ihren Unterseiten miteinander verbunden, so dass die LEDs 34 36 in unterschiedliche Richtungen scheinen können.

Der zweiten Kanal 8 ist im Bereich des zweiten Schlauchendes 14 geschlossen ausgeführt. Der erste Kanal 6 weist am zweiten Schlauchende 14 Öffnungen 22 in der Außenwand 4 des Hohlkörpers 2 auf. Ferner weist der erste Kanal 6 eine Öffnung 18 auf, die in dem Abschlusselement 16 vorgesehen ist und in den Kanal 6 mündet.

Das Abschlusselement 16 ist aus einem Material 20 gebildet, das vorzugsweise Bariumsulfat aufweist und somit durch Röntgenverfahren erkennbar ist.

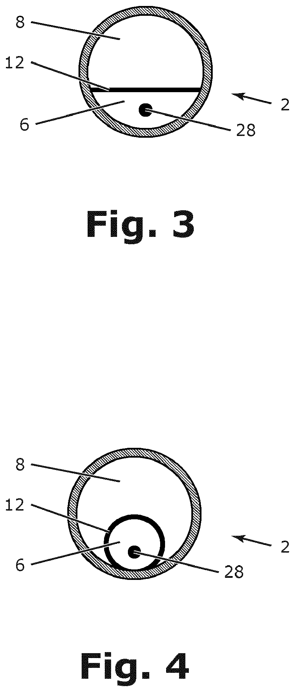

In Fig. 3 ist ein Querschnitt durch den Hohlkörper 2 dargestellt. In diesem Querschnitt ist der erste Kanal 6 und der zweite Kanal 8 zu erkennen sowie das durch den Kanal 8 geführte Stromkabel 28.

Die Außenwand 4 des Hohlkörpers 2 weist vorzugsweise einen runden Querschnitt auf. Die Außenwand 4 könnte jedoch auch jegliche andere Form aufweisen. Der runde Querschnitt der Außenwand 4 hat jedoch den Vorteil, dass, wenn der Kalibrierschlauch 1 in den Körper einführt wird, kann die Außenwand 4 des Hohlkörpers 2 nirgendwo hängenbleiben und somit keine Verletzungen im Körper verursacht.

Die Außenwand 4 des Hohlkörpers 2 sowie die Begrenzungswand 12 weisen vorzugsweise ein Material auf, das Silikon aufweist. Besonders bevorzugt besteht die Außenwand 4 des Hohlkörpers 2 sowie die Begrenzungswand 12 aus Silikon. Auch das Abschlusselement 16 kann ein Material aufweisen, das Silikon aufweist.

Der Hohlkörper 4 und die Begrenzungswand 12 bestehen aus einem solchen Material, dass sichergestellt ist, dass der Hohlkörper flexibel ist und somit leicht gebogen werden kann. Auf diese Weise lässt sich der Hohlkörper leicht durch die Speiseröhre in den Magen einführen.

In Fig. 4 ist ein alternatives Ausführungsbeispiel dargestellt, bei der die Begrenzungswand 12 anders ausgeführt ist als in Fig. 3. Ansonsten ist das Ausführungsbeispiel genauso aufgebaut wie das Ausführungsbeispiel nach Fign. 1-3. Die Außenwand 4 des Hohlkörpers 2 ist rund ausgeführt.

Als weiteres Ausführungsbeispiel kann ein nicht dargestellter Kalibrierschlauch 1 vorgesehen sein, der so ähnlich ausgeführt ist, wie der Kalibrierschlauch 1 gemäß Fign. 1 bis 3, jedoch mit dem Zusatz, dass in dem Hohlkörper 2 ein dritter Kanal vorgesehen ist. In dem dritten Kanal kann beispielsweise eine Seil oder Draht oder Ähnliches vorgesehen sein und am zweiten Ende des Hohlkörpers 2 mit diesem verbunden sein. Das Seil kann dann am ersten Schlauchende mittels einer Seilzugvorrichtung gezogen werden und das Seil verkürzt werden. Dadurch, dass das Seil am zweiten Schlauchende befestigt ist, kann sich der Kalibrierschlauch 1 biegen.

Fig. 5 zeigt die Stromversorgungsvorrichtung 26 gemäß Fig. 1 und 2 mit Klippverbindungen 66, mit denen die Stromversorgungsvorrichtung 26 lösbar Klippverbindung 66 mit dem Hohlkörper 2 verbunden wird. Die Klippverbindung 66 weist eine Art U-Form auf mit einem runden Grundelement 68 und zwei abstehenden Klammerbeinen 70, wobei die Klammerbeine 70 beim Aufstecken auf den Kalibrierschlauch 1 unter Aufbringung einer Kraft auseinander gedrückt werden müssen. Durch die Spannung in den Klippverbindungen 66 drücken sich die Klammerbeine 70 nach dem Aufstecken auf den Kalibrierschlauch wieder zusammen. Das Grundelement 68 weist eine solchen Innendurchmesser auf, der dem Außendurchmesser des Kalibrierschlauchs 1 entspricht. Auf diese Weise kann der Kalibrierschlauch einfach und schnell mit der Stromversorgungsvorrichtung 26 verbunden werden.

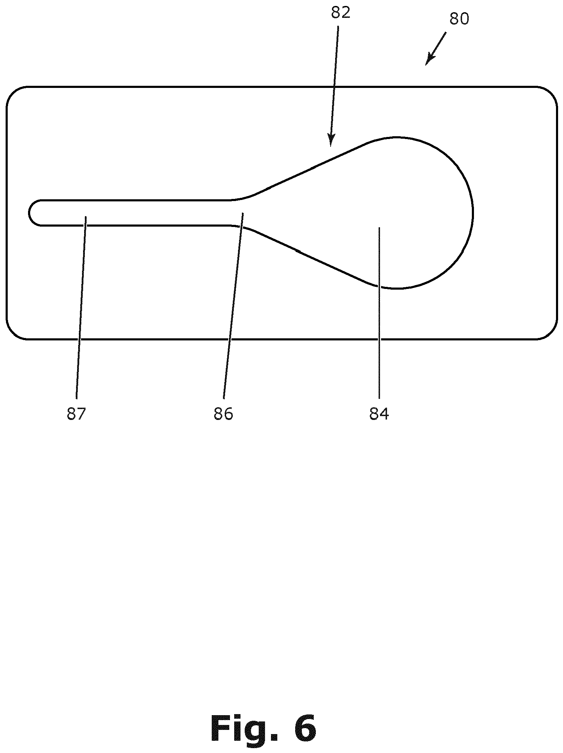

In Fig. 6 ist der Verschluss-Schieber 80 näher dargestellt. Dieser weist eine Durchtrittsöffnung 82 auf, die eine ersten Bereich 84 aufweist, durch die der Kalibrierschlauch 1 geführt werden kann ohne geklemmt zu werden. Die Durchtrittsöffnung 82 weist auf einer ersten Seite einen verjüngenden Bereich 86 auf der in eine Durchtrittskanal 87 mündet. Wenn der Kalibrierschlauch 1 in die Richtung der ersten Bereichs 86 in den Durchtrittskanal 87 gedrückt wird, wird der Kalibrierschlauch 1 zusammengedrückt und zumindest ein Kanal 6 oder auch alle Kanäle 6, 8 des Kalibrierschlauch werden verschlossen.

## Patentansprüche

1. Gastrische Kalibrierschläuche (1) mit
einem länglichen flexiblen Hohlkörper (2), der eine Außenwand (4) und ein erstes und ein zweites Schlauchende (7, 14) aufweist,
**dadurch gekennzeichnet,**
**dass** der längliche flexible Hohlkörper (2) zumindest zwei Kanäle (6, 8) aufweist, die sich jeweils in Längsrichtung des Hohlkörpers (2) erstrecken, wobei der erste Kanal (6) im Bereich des zweiten Schlauchendes (14) zumindest eine Öffnung (22, 18) aufweist und wobei der zweiten Kanal (8) am zweiten Schlauchende (14) geschlossen ist, wobei zumindest ein Leuchtmittel (30, 32) im Bereich des zweiten Schlauendes (14) im zweiten Kanal (8) angeordnet ist.

2. Gastrische Kalibrierschläuche (1) nach Anspruch 1, **dadurch gekennzeichnet, dass** eine Stromversorgungsvorrichtung (26) für das zumindest eine Leuchtmittel (30, 32) vorgesehen ist, wobei die Stromversorgungseinrichtung (26) mit dem Leuchtmittel (30, 32) über zumindest ein Stromkabel (27) verbunden ist, wobei die Stromversorgungsvorrichtung (26) außerhalb des Hohlkörpers (2) angeordnet ist.

3. Gastrische Kalibrierschläuche (1) nach Anspruch 2, **dadurch gekennzeichnet, dass** die Stromversorgungsvorrichtung (26) von außen an der Außenwand (4) des Hohlkörpers (2) mit dem Hohlkörper (2) verbunden ist.

4. Gastrische Kalibrierschläuche (1) nach einem der Ansprüche 1 bis 3,
**dadurch gekennzeichnet, dass** die Stromversorgungsvorrichtung (26) an dem Hohlkörper (2) lösbar befestigt ist, vorzugsweise mittels einer Klippverbindung mit dem Hohlkörper(2) befestigt ist.

5. Gastrische Kalibrierschläuche (1) nach einem der Ansprüche 2 bis 4,
**dadurch gekennzeichnet, dass** der zweite Kanal (8) zumindest teilweise durch die Außenwand (4) des Hohlkörpers (2) begrenzt ist, wobei die den zweiten Kanal (8) begrenzende Außenwand (4) im Bereich des ersten Schlauchendes (14) eine Austrittsöffnung (24) aufweist, durch die das zumindest eine Stromkabel (27) geführt ist, das die Stromversorgungseinrichtung (26) und das Leuchtmittel (30, 32) verbindet.

6. Gastrische Kalibrierschläuche (1) nach einem der Ansprüche 1 bis 5,
**dadurch gekennzeichnet, dass** das zumindest eine Leuchtmittel (30) ein erstes LED Band ist, das eine Oberseite und eine Unterseite aufweist, wobei auf der Oberseite mehrere nebeneinander angeordnete LEDs (34) angeordnet sind.

7. Gastrische Kalibrierschläuche (1) nach Anspruch 6, **dadurch gekennzeichnet, dass** ein zweites Leuchtmittel (32) vorgesehen ist, das ein zweites LED Band ist, das eine Oberseite und eine Unterseite aufweist, wobei auf der Oberseite mehrere nebeneinander angeordnete LEDs (34) angeordnet sind und wobei die Unterseite des zweiten LED Bandes mit der Unterseite des ersten LED Bandes verbunden ist, so dass die auf den jeweiligen Oberseiten angeordneten LEDs (32, 34) in entgegengesetzte Richtungen leuchten.

8. Gastrische Kalibrierschläuche (1) nach Anspruch 6, **dadurch gekennzeichnet, dass** auf der Unterseite des ersten LED Bandes ebenfalls mehrere nebeneinander angeordnete LEDs vorgesehen sind.

9. Gastrische Kalibrierschläuche (1) nach einem der Ansprüche 1 bis 8,
**dadurch gekennzeichnet, dass** erste und/oder zweite LED Band zwischen 2 und 30 cm lang ist.

10. Gastrische Kalibrierschläuche (1) nach einem der Ansprüche 1 bis 9,
**dadurch gekennzeichnet, dass** am zweiten Schlauchende (14) ein Abschlusselement (16) vorgesehen ist, das den Hohlkörper (2) abschließt.

11. Gastrische Kalibrierschläuche (1) nach einem der Ansprüche 1 bis 10,
**dadurch gekennzeichnet, dass** das Abschlusselement (16) stumpf ausgebildet ist.

12. Gastrische Kalibrierschläuche (1) nach einem der Ansprüche 1 bis 11,
**dadurch gekennzeichnet, dass** das Abschlusselement (16) ein Material aufweist, das radiopakes Material, vorzugsweise Bariumsulfat umfasst.

13. Gastrische Kalibrierschläuche (1) nach einem der Ansprüche 1 bis 12,
**dadurch gekennzeichnet, dass** die Stromversorgungsvorrichtung (26) an und ausschaltbar ist.

14. Gastrische Kalibrierschläuche (1) nach einem der Ansprüche 1 bis 13,
**dadurch gekennzeichnet, dass** die Stromversorgungseinrichtung (26) zumindest eine Batterie und/oder Akku aufweist.

15. Gastrische Kalibrierschläuche (1) nach einem der Ansprüche 1 bis 14,
**dadurch gekennzeichnet, dass** die Außenwand (4) und/oder Begrenzungswand () zwischen den zumindest zwei Kanälen (6, 8) aus einem Material besteht, das Silikon umfasst.
